**Europäisches Patentamt**

**European Patent Office**

**Office européen des brevets**

(19)

(11) Veröffentlichungsnummer : **0 101 879 B2**

(12) ## NEUE EUROPÄISCHE PATENTSCHRIFT

(45) Veröffentlichungstag der neuen Patentschrift :
**10.11.93 Patentblatt 93/45**

(51) Int. Cl.⁵ : **A61K 47/00, A61K 31/48**

(21) Anmeldenummer : **83107034.7**

(22) Anmeldetag : **18.07.83**

(54) **Stabile Lösungen von Mutterkornalkaloiden.**

Die Akte enthält technische Angaben, die nach dem Eingang der Anmeldung eingereicht wurden und die nicht in dieser Patentschrift enthalten sind.

(30) Priorität : **20.07.82 DE 3227122**

(43) Veröffentlichungstag der Anmeldung :
**07.03.84 Patentblatt 84/10**

(45) Bekanntmachung des Hinweises auf die Patenterteilung :
**11.01.89 Patentblatt 89/02**

(45) Bekanntmachung des Hinweises auf die Entscheidung über den Einspruch :
**10.11.93 Patentblatt 93/45**

(84) Benannte Vertragsstaaten :
**AT BE CH DE FR GB LI LU NL**

(56) Entgegenhaltungen :
**AT-A- 364 463
BE-A- 880 542
DD-A- 43 402
DD-A- 125 597**

(56) Entgegenhaltungen :
**DE-A- 2 735 587
DE-A- 2 945 636
FR-A- 2 313 059
CHEMICAL ABSTRACTS, Band 64, Nr. 10, 9. Mai 1966, Spalte 14040f, Columbus, Ohio, US; & DD - A - 43 402 (H. SCHLEGEL et al.) 25.11.1965
Pharma Acta Helvetiae. 1961 pp. 472-488**

(73) Patentinhaber : **Dr. Rentschler Arzneimittel GmbH & Co.
Postfach 320 Mittelstrasse 18
D-88471 Laupheim (DE)**

(72) Erfinder : **Köhne, Hans, Dr.
Rotbachweg 7
D-7959 Obersulmetingen (DE)**
Erfinder : **Lahr, Wolfgang
Silcherweg 29
D-7958 Laupheim 1 (DE)**
Erfinder : **Schmersahl, Hein Uwe, Dr.
Friedhofweg 6
D-7930 Ehingen 17 Gamerschwang (DE)**

(74) Vertreter : **Sandmair, Kurt, Dr. et al
Patentanwälte, Schwabe, Sandmair, Marx,
Postfach 86 02 45
D-81629 München (DE)**

EP 0 101 879 B2

## Beschreibung

Die Erfindung betrifft stabile Lösungen von Mutterkornalkaloiden und deren hydrierten Formen sowie ihrer Salze in pharmakologisch unbedenklichen Lösungsmitteln bzw. Lösungsmittelgemischen, die frei von Ethylalkohol und stabilisierenden Additiven sind, wobei als Lösungsmittelgemisch Wasser mit einem oder mehreren mehrwertigen Alkoholen verwendet wird und wobei während der Herstellung der Lösung auf eine Schutzbegasung verzichtet worden ist. Als wichtigste Vertreter der hydrierten Mutterkornalkaloide sollen Dihydroergocristin. Dihydroergokryptin, Dihydroergocornin, deren Gemische und Dihydroergotamin besonders hervorgehoben werden.

Die Instabilität dieser Alkaloide, besonders in Lösungen, führt zu erheblichen Schwierigkeiten bei der Herstellung pharmazeutisch brauchbarer flüssiger Darreichungsformen, da es im Verlauf der Lagerung infolge von Umlagerungs- und Oxidationsreaktionen zu Wirkstoffverlusten kommt.

Um diese Zersetzungserscheinungen zu verhindern, wurden verschiedene Vorschläge zur Stabilisierung der Mutterkornalkaloide gemacht.

Neben der allgemein üblichen Schutzbegasung mit inerten Gasen bei der Herstellung und Abfüllung derartiger Lösungen, wird in der DE-OS 2 555 481 vorgeschlagen. Lösungsmittelkombinationen aus Ethylalkohol, Propylenglykol, Glycerin und maximal 40 % Wasser zu verwenden.

Hartmann et al., Arznelm.-Forsch./Drug Res. 27 (II), Heft 12, 1977 schlägt ebenfalls vorgenannte Lösungsmittelkombinationen vor, wobei zur Beurteilung der Stabilität der Mutterkornalkaloide die Dielektrizitätskonstante der Lösungsmittelgemische herangezogen wird.

Die DE-OS 2 735 587 beschreibt gleichermaßen die Verwendung dieser Lösungsmittel mit einem maximalen Wassergehalt von 40 % unter Einhaltung einer Dielektrizitätskonstanten der Gemische von 30 bis 46.

Die französische Patentanmeldung 2 313 059 beschreibt zur Stabilisierung von Mutterkornalkaloiden die Verwendung von Ethylalkohol im Gemisch mit einem oder mehreren mehrwertigen Alkoholen.

Die BE-PS 880 542 beschreibt gleichfalls die Herstellung stabiler Lösungen von Mutterkornalkaloiden unter Verwendung von ein- und/oder mehrwertiger Aklohole, wobei im Beispiel 3 eine ethylalkoholfreie Lösung beschrieben wird, deren stabilisierendes Prinzip jedoch auf der notwendigen Verwendung von Stabilisatoren, wie EDTA und Propylgallat beruht.

Kennzeichnend für den Stand der Technik ist, daß zur Stabilisierung von Mutterkornalkaloiden in Lösung Ethylalkohol als notwendiger Bestandteil in Kombination mit mehrwertigen Alkoholen benötigt wird, was als schwerwiegendster Nachteil aller dieser bekannten Vorschläge angesehen werden muß, da hierdurch bestimmte Risikogruppen, nämlich Leberkrante, Alkoholkranke, Epileptiker, Hirngeschädigte, Schwangere und Kinder, von der Therapie mit Mutterkornalkaloiden in flüssiger Form als Dauertherapeutikum, ausgeschlossen sind. Diese Risikogruppen sind durch die Einnahme von Ethylalkohol gefährdet (Entwurf einer Arzneimittel-Warnhinweisverordnung des Bundesministers für Jugend, Familie und Gesundheit vom 8. April 1982).

Wegen der großer Bedeutung der Mutterkornalkaloide besonders in der Kreislauftherapie besteht somit ein anhaltendes Bedürfnis nach flüssigen Arzneiformen, die frei von Ethylalkohol als stabilisierender Komponente, über längere Zeit, auch nach Anbruch des Behältnisses, ihren Wirkstoffgehalt unverändert beibehalten.

Es war nach dem Stand der Technik nicht zu erwarten, daß Mutterkornalkaloide in Lösung unter Verzicht auf Ethylalkohol und stabilisierende Additive stabil bleiben. Es wurde nuhmehr überraschend gefunden, daß die Herstellung derartiger stabiler Lösungen jedoch möglich ist, wenn man die Mutterkornalkaloide in einem Gemisch aus Wasser sowie 1,2-Propandiol und Glycerin im Verhältnis Wasser:Aklohole von 1:4 bis 4:1 löst, wobei 1,2-Propandiol und Glycerin in einem Verhältnis von 20:1 bis 1:1 stehen. Bevorzugtes Verhältnis 1,2-Propandiol:Glycerin ist 15:1 bis 2:1. Darüber hinaus sind Polyalkohole wie beispielsweise Polyäthylenglykole verwendbar.

Es wurde weiter überraschend festgestellt, daß diese Lösungen ohne jegliche Schutzbegasung hergestellt und abgefüllt werden können und dennoch ihren Wirkstoffgehalt unverändert beibehalten, wenn man sie zwischen Raumtemperatur und 40 °C lagert.

Es hat sich ferner gezeigt, daß man bei den erfindungsgemäßen Lösungen hinsichtlich der Dielektrizitätskonstanten unabhängig ist.

Nachfolgend werden beispielhaft einige erfindungsgemäße Zusammensetzungen in Gewichtstellen genannt:

| | 1 | 2 |
|---|---|---|
| Dihydroergotaminmesilat | 20,0 | - |
| Dihydroergotoxinmesilat*) | - | 10,0 |
| Glycerin, wasserfrei | 1430,0 | 425,0 |
| 1,2-Propandiol | 4000,0 | 5300,0 |
| Wasser | 4550,0 | 4265,0 |

*) = Dihydroergotoxinmesilat stellt ein Gemisch von Dihydro-, ergocornin-, Dihydroergocristin- und Dihydroergokryptin-methansulfonat im Verhältnis 1 : 1 : 1 dar

Herstellung der Lösungen nach Beispiel 1-4

Das Wasser und Glycerin sowie 1,2-Propandiol werden miteinander gemischt. Das Alkaloid oder die Alkaloide werden zugegeben. Es wird so lange gerührt, bis eine klare Lösung entstanden ist. Nach Filtration werden die Lösungen in Fläschchen abgefüllt.

Aus nachstehender Tabelle ist ersichtlich, daß die Gehalte der Mutterkornalkaloide, ausgedrückt in Prozent, in den erfindungsgemäß hergestellten Lösungen nach längerer Lagerzeit auch bei erhöhten Temperaturen praktisch unverändert sind.

| Lagerzeit/ Monate | Beispiel 1 | | | Beispiel 2 | | |
|---|---|---|---|---|---|---|
| | RT* % | 30°C % | 40°C % | RT* % | 30°C % | 40°C % |
| 0 | 100 | 100 | 100 | 100 | 100 | 100 |
| 2 | 104 | 102,5 | 102 | 97,1 | 96,2 | 96,2 |
| 4 | 99,5 | 99 | 100 | 99 | 99 | 101 |
| 6 | - | - | - | 99 | 99 | 99 |
| 9 | - | - | - | 99,2 | 100,7 | 100 |
| 12 | - | - | - | 104 | 103 | 101 |
| 16 | - | - | 95,9 | - | - | - |

*RT = Raumtemperatur.

## Patentansprüche

1. Stabile Lösung von Mutterkornalkaloiden und deren hydrierten Formen sowie von ihren Salzen, enthaltend ein Lösungsmittelgemisch aus Wasser und mehrwertigen Alkohlen, dadurch gekennzeichnet, daß die Lösung frei von Ethylalkohol und stabilisierenden Additiven ist und bei ihrer Herstellung auf eine Schutzbegasung verzichtet worden ist und daß die Lösung als Lösungsmittelgemisch Wasser sowie 1,2-Propandiol und Glycerin im Verhältnis Wasser : Alkohole von 1 : 4 bis 4 : 1 enthält, wobei 1,2-Propandiol und Glycerin in einem Verhältnis von 20 : 1 bis 1 : 1 stehen.

2. Lösung nach Anspruch 1, dadurch gekennzeichnet, daß sie darüberhinaus Polyalkylenglykole, vorzugsweise Polyethylenglykol enthält.

3. Lösung nach Anspruch 1 und Anspruch 2, dadurch gekennzeichnet, daß sie als Mutterkornalkaloid Dihydroergocristin oder ein Salz davon enthält.

4. Lösung nach Anspruch 1 und Anspruch 2, dadurch gekennzeichnet, daß sie als Mutterkornalkaloid ein Gemisch aus Dihydroergocornin, Dihydroergocristin und Dihydroergokryptin oder eines der Salze davon, vorzugsweise im Verhältnis 1 : 1 : 1, enthält.

5. Lösung nach Anspruch 1 und Anspruch 2, dadurch gekennzeichnet, daß sie als Mutterkornalkaloid Dihydroergotamin oder ein Salz davon enthält.

## Claims

1. Stable solution of ergot alkaloids and their hydrated forms, as well as their salts, containing a mixture of solvents of water and polyhydric alcohols characterised in that this solution is free of ethyl alcohol and stabilizing additives and in that during the manufacture of the solution a protective gasification has not been used and in that the solution contains as a solvent mixture water and 1,2-propane-diol and glycerol in a ratio of water : alcohols from 1 : 4 to 4 : 1, the ratio of 1,2-propanediol and glycerol being from 20 : 1 to 1 : 1.

2. Solution according to claim 1, characterised in that the solution in a addition contains polyalkylene glycols, preferably polyethylene glycol.

3. Solution according to claims 1 and 2, characterised in that the solution contains as ergot alkaloid dihydro-ergocristine or its salts.

4. Solution according to claims 1 to 2, characterised in that the solution contains as ergot alkaloid a mixture of dihydro-ergocornine, dihydro-ergocristine and dihydro-ergocryptine or their salts, preferably in the ratio 1 : 1 : 1.

5. Solution according to claims 1 to 2, characterised in that the solution contains as ergot alkaloid dihydroergotamine or a salts thereof.

## Revendications

1. Solution stable d'alcaloides de l'ergot et de leurs formes hydrogénées ainsi que de leurs sels, contenant un mélange de solvants constitué de l'eau et d'alcools plurifonctionnels, caractérisées en ce qu' la solution est exempts d'alcool éthylique et d'additifs stabilisants et la fabrication de cette solution étant effectuée sans recourir à une atmosphère protectrice, et la solution comme mélange de solvants contenant l'eau et 1,2-propanediol et de la glycérine dans un rapport de 1 : 4 à 4 : 1, 1,2-propanediol et de la glycérine sont présents dans un rapport de 20 : 1 à 1 : 1.

2. Solution selon la revendication 1, caractérisées en ce que des polyalkylèneglycols comme le polyéthylèneglycol peuvent être utilisés en plus.

3. Solution selon les revendications 1 et 2, caractérisées en ce qu'elles contiennent comme alcaloide de l'ergot la dihydroergocristine ou ses sels.

4. Solution selon les revendications 1 à 2, caractérisées en ce qu'elles contiennent comme alcaloide de l'ergot un mélange de la dihydroergocornine, de la dihydroergocristine et de la dihydroergokryptine ou de leurs sels, de préférence dans un rapport de 1 : 1 : 1.

5. Solution selon les revendications 1 à 2, caractérisées en ce qu'elles contiennent comme alcaloide de l'ergot la dihydroergotamine ou ses sels.